# EUROPEAN PATENT APPLICATION

(11) **EP 3 711 746 A1**
(43) Date of publication of application: **23.09.2020**
(21) Application number: 18878790.7
(22) Date of filing: 30.10.2018
(51) Int. Cl.: A61K 8/70, A61K 8/02, A61K 8/31, A61K 8/37, A61K 8/64, A61K 8/891, A61Q 19/08

(54) **OIL-BASED COMPOSITION**

(30) Priority: 15.11.2017 JP 2017220078
(71) Applicant: Pola Chemical Industries Inc., Fukuroi-shi Shizuoka 437-8765 (JP)
(72) Inventor: DAIRAKU, Sachiko, Yokohama-shi Kanagawa 244-0812 (JP); TAKEUCHI, Satomi, Yokohama-shi Kanagawa 244-0812 (JP)
(74) Representative: Jones, Nicholas Andrew
(86) International application number: PCT/JP2018/040287
(87) International publication number: WO 2019/098010

(57) **Abstract**

Provided is a method for blending a compound effective in preventing or improving skin aging with a composition in a chemically stable manner. Also provided is a method for increasing the uniform dispersibility of the compound in the composition. A compound represented by the following general formula (1), an isomer thereof, and/or a pharmacologically acceptable salt thereof is dispersed in an oiling agent having 0 to 2 hydroxy groups to prepare an oil-based composition, thereby suppressing the decomposition of the compound. Moreover, the composition further contains a powder, a solid fat and/or a semi-solid fat, thereby suppressing the sedimentation of the compound in the composition over time. 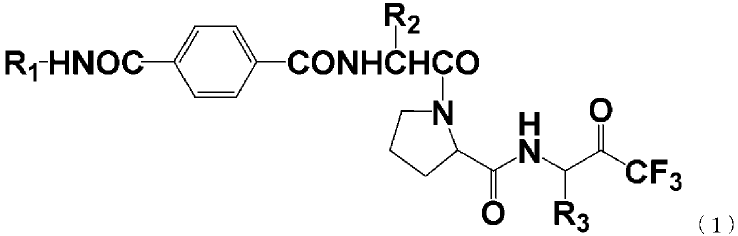 [In formula (1), R₁ represents a linear or branched C1-4 alkyl group substituted with a carboxyl group or a linear or branched C1-4 alkyl group substituted with a carboxylic acid ester group having a C1-4 alkyl chain, and R₂ and R₃ each independently represent a linear or branched C1-4 alkyl group.]

## Description

### TECHNICAL FIELD

The present invention relates to an oil-based composition stably comprising an active component of an anti-aging cosmetic.

### BACKGROUND ART

A typical symptom of skin aging phenomena due to aging is wrinkle formation. Wrinkles are roughly classified into shallow wrinkles formed on the skin surface, which can be improved by moisturizing, and deep wrinkles caused by exposure to ultraviolet light and accumulation of physical irritations. Prevention or improvement of the latter deep wrinkle formation is very difficult. Further, various wrinkle-improving agents have been developed to date (see, for example, Non-Patent Document 1). As such wrinkle-improving agents, those containing retinoic acid as an active component are famous. Retinoic acid has been approved as a therapeutic drug for wrinkles and acne in the United States, but has not been approved in Japan due to problems in terms of safety including skin irritations. Further, as other wrinkle-improving agents, collagen production promoters (see, for example, Patent Document 1), hyaluronic acid production promoters (see, for example, Patent Document 2), and the like have been reported. However, it is difficult to say that any of the above-mentioned wrinkle-improving agents is sufficiently effective, and effective means have not been established yet.

An example of wrinkle-improving agents having a mechanism of action different from that of the aforementioned wrinkle-improving agents is an elastase inhibitor. Elastin, which is one of the structural proteins present in skin tissue, builds a cross-linked structure to maintain tissue elasticity. It is known that skin irritations such as UV exposure denatures and disintegrates elastin by causing over-expression and activation of elastase, which is an elastin degrading enzyme, lowering skin firmness and elasticity, and promoting wrinkle formation. Elastase inhibitors exert a preventive or improving effect on wrinkle formation by inhibiting the aforementioned elastin degrading enzyme. On the other hand, structural analysis and structure-activity correlation studies of elastin degrading enzymes and substrates are underway, but it is difficult to realize the high enzyme inhibitory activity and selectivity of small organic molecules, and many peptides and derivatives thereof (see, for example, Patent Document 3) are present as components having high enzyme inhibitory activity.

Like these peptide derivatives known as elastase inhibitors, peptide derivatives including WS7622A mono- or di-sulfuric acid ester are known (see, for example, Patent Document 4), and application thereof to ischemic diseases using such pharmacological action has been attempted. Further, a compound represented by the following general formula (1) is known to have an effect of inhibiting leukocyte elastase like WS7622A, and has been reported to have an effect of preventing or treating skin aging (for example, see Patent Document 5). 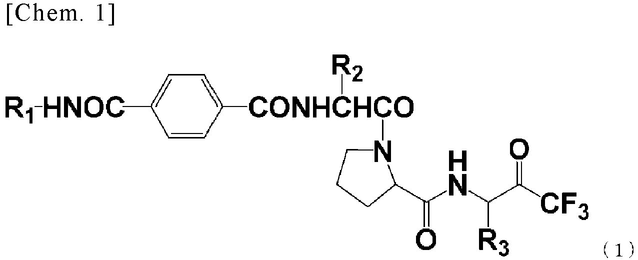 [Wherein R₁ represents a linear or branched C1-4 alkyl group substituted with a carboxyl group, or a linear or branched C1-4 alkyl group substituted with a carboxylic acid ester group having a C1-4 alkyl chain, and R₂ and R₃ each independently represent a linear or branched C1-4 alkyl group.]

### PRIOR ART REFERENCES

### Patent Documents

[Patent Document 1] Japanese Laid-open Patent Application (Kokai) No. 2002-255847
[Patent Document 2] Japanese Laid-open Patent Application (Kokai) No. 2004-123637
[Patent Document 3] International Publication No. WO2001/40263
[Patent Document 4] International Publication No. WO1998/27998
[Patent Document 5] International Publication No. WO1999/43352

### Non Patent Document

[Non Patent Document 1] Technology for development of anti-aging, whitening and moisturizing cosmetics, CMC Publishing, compiled under the supervision of Masato Suzuki, Chapter 2 Anti-aging and anti-wrinkle functional cosmetics

### DISCLOSURE OF THE INVENTION

### PROBLEMS TO BE SOLVED BY THE INVENTION

The present inventors have discovered that the compound represented by the general formula (1) is easily decomposed and thus is problematic in stability when the compound is contained in an anti-aging composition for skin.

The present invention has been achieved under such circumstances, and an object of the present invention is to provide a method for blending the compound represented by the general formula (1), an isomer thereof and/or a pharmacologically acceptable salt thereof with a composition in a chemically stable manner.

### MEANS FOR SOLVING THE PROBLEMS

In view of such circumstances, as a result of intensive studies, the present inventors have arrived at the fact that the decomposition of the compound represented by the above general formula (1) can be suppressed by dispersing the compound, an isomer thereof and/or a pharmacologically acceptable salt thereof in a specific oiling agent to give an oil-based composition.

Moreover, the dispersed components are generally precipitated over time in the composition of the dispersion system. The present inventors have also discovered that the compound represented by the above general formula (1), an isomer thereof and/or a pharmacologically acceptable salt thereof can be uniformly dispersed and dispersion stability can be maintained over time, when the composition further comprises a powder, a solid fat and/or a semi-solid fat. Thus, the present inventors have completed the present invention.

That is, the present invention is as follows.
[1] An oil-based composition, comprising a compound represented by the following general formula (1), an isomer thereof and/or a pharmacologically acceptable salt thereof and an oiling agent having 0 to 2 hydroxy groups. 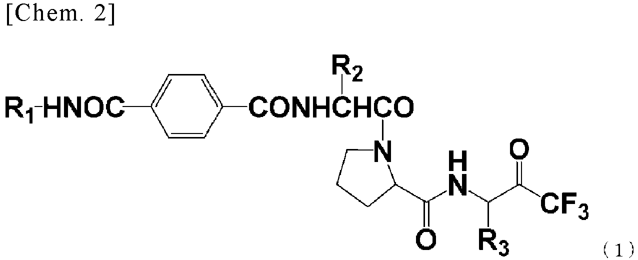 [Wherein R₁ represents a linear or branched C1-4 alkyl group substituted with a carboxyl group, or a linear or branched C1-4 alkyl group substituted with a carboxylic acid ester group having a C1-4 alkyl chain, and R₂ and R₃ each independently represent a linear or branched C1-4 alkyl group.]
[2] The composition according to [1], wherein the oiling agent having 0 to 2 hydroxy groups is selected from the group consisting of hydrocarbon oil, silicone oil, and ester oil.
[3] The composition according to [1] or [2], wherein the content of the oiling agent having 0 to 2 hydroxy groups is 50 mass% or more of the whole composition.
[4] The composition according to any one of [1] to [3], further comprising an oily gelling agent.
[5] The composition according to any one of [1] to [4], further comprising a powder.
[6] The composition according to [5], wherein the content of the powder ranges from 0.5 mass% to 20 mass% of the whole composition.
[7] The composition according to any one of [1] to [6], which is in a form of oily gelling agent.
[8] The composition according to [7], comprising a solid fat and/or a semi-solid fat in an amount of 5 mass% or more of the whole composition.
[9] The composition according to any one of [1] to [8], which substantially contains no water.
[10] The composition according to any one of [1] to [9], which is a skin external preparation.

### ADVANTAGEOUS EFFECTS OF THE INVENTION

According to the present invention, a chemically stable composition can be provided, in which the decomposition of the compound represented by the above general formula (1), an isomer thereof and/or a pharmacologically acceptable salt thereof is suppressed. Further, a composition in which the compound is uniformly dispersed over time can be provided.

### MODE FOR CARRYING OUT THE INVENTION

The composition of the present invention comprises the compound represented by the general formula (1), an isomer thereof and/or a pharmacologically acceptable salt thereof, and an oiling agent having 0 to 2 hydroxy groups.

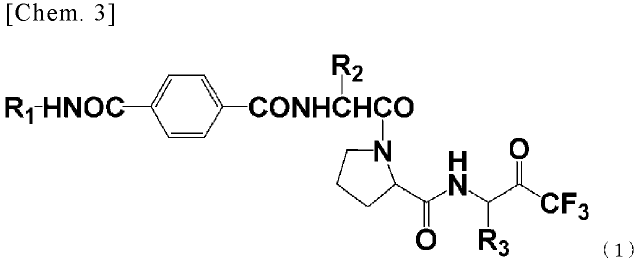

The compound represented by the above general formula (1), an isomer thereof and/or a pharmacologically acceptable salt thereof can be produced by the method described in Japanese Laid-open Patent Application (Kokai) No. H04-297446.

The compound represented by the above general formula (1) is an optically active compound having a plurality of asymmetric carbon atoms in its molecular structure, and thus enantiomers and diastereomers exist as isomers thereof. Furthermore, as the compound represented by the above general formula (1) of the present invention, a compound, in which a racemate, an enantiomer, a diastereomer, and further the above isomer are mixed in an arbitrary ratio, exists.

The compound represented by the above general formula (1) of the present invention is as described below. In the formula, R₁ represents a linear or branched C1-4 alkyl group substituted with a carboxyl group or a linear or branched C1-4 alkyl group substituted with a carboxylic acid ester group having a C1-4 alkyl chain, and R₂ and R₃ each independently represent a linear or branched C1-4 alkyl group.

Specific preferable examples of R₁ above can suitably include a carboxymethyl group, a carboxyethyl group, a carboxypropyl group, a carboxybutyl group, a methoxycarbonylmethyl group, a methoxycarbonylethyl group, a methoxycarbonylpropyl group, methoxycarbonylbutyl group, ethoxycarbonylmethyl group, ethoxycarbonylethyl group, ethoxycarbonylpropyl group, ethoxycarbonylbutyl group, propyloxycarbonylmethyl group, propyloxycarbonylethyl group, propyloxycarbonylpropyl group, propyloxycarbonylbutyl group, butyloxycarbonylmethyl group, a butyloxycarbonylethyl group, a butyloxycarbonylpropyl group and a butyloxycarbonylbutyl group. More preferably, a carboxymethyl group can be suitably illustrated.

Specific preferred examples of R₂ and R₃ above can suitably include independently a methyl group, an ethyl group, an n-propyl group, an isopropyl group, an n-butyl group, an isobutyl group, a sec-butyl group and a tert-butyl group. More preferably, an isopropyl group can be illustrated suitably.

The compound represented by the above general formula (1) has human leukocyte elastase inhibitory activity, and is expected to have a therapeutic effect on cerebral ischemic diseases including cerebral infarction. Further, for example, as described in International Publication No. WO1999/43352, the compound has a preventive or therapeutic effect on skin aging. Moreover, the compound represented by the above general formula (1) of the present invention has effects such as the generation of elastic fibers in the papillary dermis, the generation of fine collagen fibers at the dermal site just below the epidermis, and increases in epidermal thickness. With such effects, the compound exerts a preventive or improving effect on wrinkle formation.

The compound represented by the above general formula (1) is preferably a compound represented by the following general formula (2).

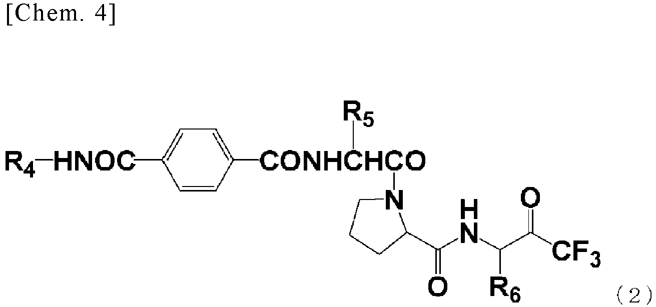

The compound represented by the above general formula (2) of the present invention is as described below. In the formula, R₄ represents a linear or branched C1-4 alkyl group substituted with a carboxyl group, R₅ and R₆ each independently represent a linear or branched C1-4 alkyl group. R₄ above represents a linear or branched C1-4 alkyl group substituted with a carboxyl group, and specific preferable examples of R₄ above can suitably include a carboxymethyl group, a 1-carboxyethyl group, a 2-carboxyethyl group, a 1-carboxypropyl group, a 2-carboxypropyl group, a 3-carboxypropyl group, a 1-carboxybutyl group, a 2-carboxybutyl group, a 3-carboxybutyl group, and a 4-carboxybutyl group. Further preferable examples thereof can suitably include a carboxymethyl group.

R₅ and R₆ above each independently represent a linear or branched C1-4 alkyl group. Specific preferable examples of R₅ and R₆ above can suitably include a methyl group, an ethyl group, an n-propyl group, an isopropyl group, an n-butyl group, an isobutyl group, a sec-butyl group, and a tert-butyl group. Further preferable examples thereof can suitably include an isopropyl group. Examples of the compound represented by the above general formula (2) can suitably include 3-[[4-(carboxymethylaminocarbonyl)phenylcarbonyl]-valyl-prolyl]amino-1,1,1-trifluoro-4-methyl-2-oxopentane, N-[4-[[(carboxymethyl)amino]carbonyl]benzoyl]-L-alanyl-N-[3,3,3-trifluoro-1-(1-methyl)-2-oxopropyl]-L-prolinamide, N-[4-[[(carboxyethyl)amino]carbonyl]benzoyl]-L-alanyl-N-[3,3,3-trifluoro-1-(1-methyl)-2-oxopropyl]-L-prolinamide, N-[4-[[(carboxypropyl)amino]carbonyl]benzoyl]-L-alanyl-N-[3,3,3-trifluoro-1-(1-methyl)-2-oxopropyl]-L-prolinamide, N-[4-[[(carboxybutyl)amino]carbonyl]benzoyl]-L-alanyl-N-[3,3,3-trifluoro-1-(1-methyl)-2-oxopropyl]-L-prolinamide, N-[4-[[(carboxymethyl)amino]carbonyl]benzoyl]-L-alanyl-N-[3,3,3-trifluoro-1-(1-ethyl)-2-oxopropyl]-L-prolinamide, N-[4-[[(carboxyethyl)amino]carbonyl]benzoyl]-L-alanyl-N-[3,3,3-trifluoro-1-(1-ethyl)-2-oxopropyl]-L-prolinamide, N-[4-[[carboxypropyl)amino]carbonyl]benzoyl]-L-alanyl-N-[3,3,3-trifluoro-1-(1-ethyl)-2-oxopropyl]-L-prolinamide, N-[4-[[(carboxybutyl)amino]carbonyl]benzoyl]-L-alanyl-N-[3,3,3-trifluoro-1-(1-ethyl)-2-oxopropyl]-L-prolinamide, N-[4-[[(carboxymethyl)amino]carbonyl]benzoyl]-L-alanyl-N-[3,3,3-trifluoro-1-(1-methylethyl)-2-oxopropyl]-L-prolinamide, N-[4-[[(carboxyethyl)amino]carbonyl]benzoyl]-L-alanyl-N-[3,3,3-trifluoro-1-(1-methylethyl)-2-oxopropyl]-L-prolinamide, N-[4-[[(carboxypropyl) amino]carbonyl]benzoyl]-L-alanyl-N-[3,3,3-trifluoro-1-(1-methylethyl)2-oxopropyl]-L-prolinamide, N-[4-[[(carboxybutyl)amino]carbonyl]benzoyl]-L-alanyl-N-[3,3,3-trifluoro-1-(1-methylethyl)-2-oxopropyl]-L-prolinamide, N-[4-[[(carboxymethyl)amino]carbonyl]benzoyl]-L-valyl-N-[3,3,3-trifluoro-1-(1-methyl) -2-oxopropyl]-L-prolinamide, N-[4-[[(carboxyethyl)amino]carbonyl]benzoyl]-L-valyl-N-[3,3,3-trifluoro-1-(1-methyl)-2-oxopropyl]-L-prolinamide, N-[4-[[(carboxypropyl)amino]carbonyl]benzoyl]-L-valyl-N-[3,3,3-trifluoro-1-(1-methyl) -2-oxopropyl]-L-prolinamide, N-[4-[[(carboxybutyl)amino]carbonyl]benzoyl]-L-valyl-N-[3,3,3-trifluoro-1-(1-methyl)-2-oxopropyl]-L-prolinamide, N-[4-[[(carboxymethyl)amino]carbonyl]benzoyl]-L-valyl-N-[3,3,3-trifluoro-1-(1-ethyl)-2-oxopropyl]-L-prolinamide, N-[4-[[(carboxyethyl)amino]carbonyl]benzoyl]-L-valyl-N-[3,3,3-trifluoro-1-(1-ethyl)-2-oxopropyl]-L-prolinamide, N-[4-[[(carboxypropyl)amino]carbonyl]benzoyl]-L-valyl-N-[3,3,3-trifluoro-1-(1-ethyl)-2-oxopropyl]-L-prolinamide, N-[4-[[(carboxybutyl)amino]carbonyl]benzoyl] -L-valyl-N-[3,3,3-trifluoro-1-(1-ethyl)-2-oxopropyl]-L-prolinamide, N-[4-[[(carboxymethyl)amino]carbonyl]benzoyl]-L-valyl-N-[3,3,3-trifluoro-1-(1-methylethyl)-2-oxopropyl]-L-prolinamide(3(RS)-N-[4-[[(carboxymethyl)amino] carbonyl]benzoyl]-L-valyl-N-[3,3,3-trifluoro-1-(1-methylethyl)-2-oxopropyl]-L-prolinamide), N-[4-[[(carboxyethyl)amino]carbonyl]benzoyl]-L-valyl-N-[3,3,3-trifluoro-1-(1-methylethyl)-2-oxopropyl]-L-prolinamide, N-[4-[[(carboxypropyl) amino]carbonyl]benzoyl]-L-valyl-N-[3,3,3-trifluoro-1-(1-methylethyl)-2-oxopropyl]-L-prolinamide, N-[4-[[(carboxybutyl)amino]carbonyl]benzoyl]-L-valyl-N-[3,3,3-trifluoro-1-(1-methylethyl)-2-oxopropyl]-L-prolinamide.

Among the compounds represented by the above general formula (2), a more preferable example thereof is 3-[[4-(carboxymethylaminocarbonyl)phenylcarbonyl]-valyl-prolyl]amino-1,1,1-trifluoro-4-methyl-2-oxopentane, and a further preferable example thereof is 3(RS)-[[4-(carboxymethylaminocarbonyl)phenylcarbonyl]-L-valyl-L-prolyl]amino-1,1,1-trifluoro-4-methyl-2-oxopentane represented by the following formula (3). Further, as a compound to be contained in the composition of the present invention, a sodium salt of the above 3(RS)-[[4-(carboxymethylaminocarbonyl)phenylcarbonyl]-L-valyl-L-prolyl]amino-1,1,1-trifluoro-4-methyl-2-oxopentane (hereinafter also referred to as "KSK32") is particularly preferred.

In another nomenclature, KSK32 is also denoted as N-[4-[[(carboxymethyl)amino]carbonyl]benzoyl]-L-valyl-N-[(RS)-3,3,3-trifluoro-1-(1-methylethyl)-2-oxopropyl]-L-prolinamide, but is the same compound as KSK32.

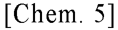

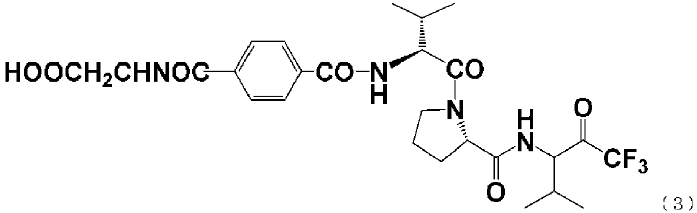

In the present invention, the compound represented by the above general formula (1) can be contained as it is in the composition, but it can be treated with a pharmacologically acceptable acid or base to convert it into a salt form, and then used as a salt. Examples thereof include inorganic acid salts such as hydrochloride, sulfate, nitrate, phosphate and carbonate; organic acid salts such as maleate, fumarate, oxalate, citrate, lactate, tartrate, methanesulfonate, para-toluene sulfonate and benzene sulfonate; alkali metal salts such as sodium salt and potassium salt; alkaline earth metal salts such as calcium salt and magnesium salt; organic amine salts such as triethylamine salt, triethanolamine salt, ammonium salt, monoethanolamine salt, and piperidine salt; and basic amino acid salts such as lysine salt and arginate.

Further, the composition of the present invention can contain one type or two or more types selected from the compound represented by the above general formula (1), an isomer thereof and/or a pharmacologically acceptable salt thereof.

The content of the compound represented by the above general formula (1), an isomer thereof and/or a pharmacologically acceptable salt thereof in the composition of the present invention is not particularly limited and ranges from preferably 0.01 mass% to 10 mass%, and more preferably 0.1 mass% to 5 mass% relative to the whole composition. The composition contains the component within such a range, so that the compound represented by the above general formula (1), an isomer thereof and/or a pharmacologically acceptable salt thereof can easily exhibit an effect of preventing or improving wrinkle formation or will be able to sufficiently exhibit the effect.

The composition of the present invention is prepared by dispersing the compound represented by the above general formula (1), an isomer thereof and/or a pharmacologically acceptable salt thereof in an oiling agent having 0 to 2 hydroxy groups.

Such an oiling agent is not particularly limited, and is selected from the group consisting of hydrocarbon oil, silicone oil, and ester oil. Further, the number of hydroxy groups more preferably ranges from 0 to 1, and it is more preferable that the number of hydroxy groups is 0, that is, no hydroxyl group is contained.

Here, the number of hydroxy groups means the number thereof per molecule.

Preferred examples of the hydrocarbon oil include paraffin, isoparaffin, liquid paraffin, α-olefin oligomer, polyethylene, squalane, isododecane, and isohexadecane.

The silicone oil may or may not have a side chain, and may or may not be crosslinked. Preferred examples of the silicone oil include polysiloxane, dimethylpolysiloxane, decamethylcyclopentasiloxane, methylpolysiloxane and diphenyl dimethicone.

Preferred examples of ester oil include glyceryl tri-2-ethylhexanoate, glyceryl triisostearate, cetyl 2-ethylhexenoate, glyceryl tri(capryl/capric acid), ethyleneglycol distearate, N-lauroyl sarcosine, glyceryl triisostearate, diisostearyl malate, and ethylene glycol monostearate.

In the composition of the present invention, an oiling agent enables dispersion of the compound represented by the above general formula (1), an isomer thereof and/or a pharmacologically acceptable salt thereof in the oiling agent.

Further, the compound represented by the above general formula (1), an isomer thereof and/or a pharmacologically acceptable salt thereof is easily decomposed. Accordingly, there is a problem such that in a composition containing the component, the content of the compound decreases over time. With respect to this problem, the present invention involves the use of an oiling agent having 0 to 2 hydroxy groups as a dispersion medium, so as to allow the compound to be chemically stably blended in the composition.

Among oiling agents to be used for the composition of the present invention, the content of the oiling agent having 0 to 2 hydroxy groups is preferably 50 mass% or more, more preferably 80 mass% or more, and further preferably 90 mass% or more relative to the whole composition. The content of the oiling agent within such a range facilitates the dispersion of the compound represented by the above general formula (1) an isomer thereof and/or a pharmacologically acceptable salt thereof.

Further, the total content of an oiling agent in the composition of the present invention is generally 50 mass% or more and preferably 60 mass% or more relative to the whole composition.

Moreover, the content of an oiling agent having 3 or more hydroxy groups is preferably 50 mass% or less, more preferably 25 mass% or less, and further preferably 5 mass% or less relative to the whole composition. This is because when such an oiling agent having 3 or more hydroxy groups coexists with other components at least a given level, the compound represented by the above general formula (1), an isomer thereof and/or a pharmacologically acceptable salt thereof will be easily decomposed and the residual amount of the compound in the composition may decrease with time.

Here, examples of the oiling agent having a hydroxy group(s) include, but are not limited to, a mono- or di-ester of fatty acid and polyglycerin, an ester of fatty acid and sugar alcohol, and an ester of fatty acid and polysaccharide alcohol.

Further, the oiling agent in the composition of the present invention contains preferably a solid fat and/or a semisolid fat, more preferably a solid fat and/or a semisolid fat having 0 to 2 hydroxy groups, further preferably a solid fat and/or a semisolid fat having no hydroxy group.

The composition contains a solid fat or a semi-solid fat, so that the viscosity of the composition is increased. This hinders the sedimentation of the compound represented by the above general formula (1), an isomer thereof and/or a pharmacologically acceptable thereof dispersed in the composition even after the lapse of time. The resulting composition can be excellent in dispersion stability over time. Such an embodiment is suitable for the oily gelling agent form etc., described later.

The total content of a solid fat and/or a semi-solid fat is preferably 5 mass% or more, more preferably 10 mass%, and further preferably 20 mass% or more of the whole composition. Moreover, 50 mass% or less of the whole composition is preferable, 40 mass% or less of the same is more preferable, and 30 mass% or less of the same is further preferable.

Here, examples of a solid fat also include a semi-solid fat. The term "solid" means that the relevant substance is non-flowable at 25°C, and the term "semi-solid" means that the relevant substance is almost not deformed in an environment where no stress is present at 1 atm and 20°C, but is deformed when some stress (about 10 to 100 g/cm²) is applied. A solid fat having a melting point of 50°C or higher is more preferable.

Examples of a solid fat and/or a semi-solid fat include plant-derived fats, such as carnauba wax, Japan wax, candelilla wax, rice bran wax, shea butter, and African mango butter, and animal-derived fats such as beeswax, shellac wax and Chinese wax, petroleum-derived fats, specifically, refined wax derived from petroleum, such as paraffin wax and microcrystalline wax, and mineral-derived fats, specifically, refined wax derived from minerals, such as ozokerite, ceresin, and montan wax. However, in general, solid fats and semi-solid fats derived from animals and plants are more likely to contain a component having a hydroxy group, and thus refined waxes derived from petroleum or minerals are more preferable. In addition, these solid fats and/or semi-solid fats can be used individually or in combination of 2 or more types thereof.

The composition of the present invention preferably further contains an oily gelling agent. The composition contains an oily gelling agent, so that the viscosity of the composition is increased. Accordingly, sedimentation of the compound represented by the above general formula (1), an isomer thereof and/or a pharmacologically acceptable salt thereof dispersed in the composition is difficult over time, so that the composition can be excellent in dispersion stability over time.

Here, the term "oily gelling agent" refers to a gelling agent compatible with an oily component such as an oiling agent. Examples thereof can include, but are not particularly limited to, 12-hydroxystearic acid, dextrin palmitate, dextrin (palmitate/octanoate), dibutyllauroyl glutamide, and dimethicone crosspolymer.

The content of an oily gelling agent is preferably 0.5 mass% or more, more preferably 2 mass%, and further preferably 5 mass% or more of the whole composition. Moreover, 20 mass% or less is preferable, 15 mass% or less is more preferable, and 10 mass% or less of the whole composition is further preferable.

When the composition of the present invention is in the oily gelling agent form described later, the composition preferably contains an oily gelling agent in addition to or instead of the above solid fat or semi-solid fat. The content in this case, specifically, the total content of the solid fat and the semi-solid fat is preferably 5 mass% or more of the whole composition.

The composition of the present invention further preferably contains a powder. The composition contains a powder, so that the viscosity of the composition is increased. Accordingly, the sedimentation of the compound represented by the above general formula (1), an isomer thereof and/or a pharmacologically acceptable salt thereof dispersed in the composition is difficult over time, so that the composition can be excellent in dispersion stability over time. Such an embodiment is suitable for the oily gelling agent form and the like described later.

The content of a powder ranges from preferably 0.5 mass% to 20 mass% and more preferably 1 mass% to 5 mass% of the whole composition.

Examples of powders include powders, the surface of which may be treated, such as silica (anhydrous silicic acid), mica, talc, kaolin, synthetic mica, calcium carbonate, magnesium carbonate, magnesium oxide, aluminum oxide, and barium sulfate, inorganic dyes, the surface of which may be treated, such as iron red, yellow iron oxide, black iron oxide, cobalt oxide, ultramarine blue, iron blue, titanium oxide and zinc oxide, pearl agents, the surface of which may be treated, such as mica titanium, fish scale flake, and bismuth oxychloride, organic pigments, which may be laked, such as red No. 202, red No. 228, red No. 226, yellow No. 4, blue No. 404, yellow No. 5, red No. 505, red No. 230, red No. 223, orange No. 201, red No. 213, yellow No. 204, yellow No. 203, blue No. 1, green No. 201, purple No. 201, and red No. 204, and organic powders such as polyester powder, polyamide powder, polyethylene powder, methyl polymethacrylate, nylon powder, and organopolysiloxane elastomer.

Further, examples of the shape thereof include, but are not particularly limited to, spherical, semispherical, and plate-like shapes which are generally used for skin external preparations such as cosmetics. In addition, the powders may also be porous powders.

Moreover, the particle diameter may be a diameter that is generally used for skin external preparations, such as cosmetics.

Among these, in the present invention, a particularly preferable example thereof is porous silica in view of the dispersion stability of the composition.

The composition of the present invention is oily and usually contains substantially no water. Here, the expression "does not substantially contain" means that the content is preferably 0.1 mass% or less, more preferably 0.01 mass% or less, and further preferably 0.001 mass% or less relative to the whole composition.

When water is present in the composition, the compound represented by the above general formula (1), an isomer thereof and/or a pharmacologically acceptable salt thereof becomes easily decomposed, and the residual amount of the compound in the composition may be decreased over time.

The oil-based composition of the present invention can be prepared in any dosage form such as a form of milk, a form of cream, a form of oily gelling agent, or the like regardless of fluidity, but the form is preferably a form of oily gelling agent.

In the present invention, the oily gelling agent form is usually in a state of having high viscosity and low flowability. For example, the composition, which is non-flowable at 25°C., that is, solid, is preferable. Moreover, the composition preferably has a viscosity of 20,000 Pa/s or more at 25°C. With such a dosage form, the sedimentation of the compound represented by the above general formula (1), an isomer thereof and/or a pharmacologically acceptable salt thereof is difficult over time, and thus the composition can be excellent in dispersion stability over time.

The composition of the present invention is characterized in that the residual amount of the compound represented by the above general formula (1), an isomer thereof and/or a pharmacologically acceptable salt thereof is high over time in the composition. Specifically, for example, the content of the compound after storage for 2 weeks at 60°C is preferably 60% or more, more preferably 80% or more, further preferably 95% or more relative to the content of the compound after storage for 2 weeks at 5°C.

The composition of the present invention is characterized in that the dispersion stability in the composition over time of the compound represented by the above general formula (1), an isomer thereof and/or a pharmacologically acceptable salt thereof is high. Specifically, for example, when the composition is left to stand at 40°C for 1 day, the sedimentation of the compound is not visually recognized. Alternatively, when the composition is left to stand at 40°C for 1 day, a difference in concentration of the compound between the upper portion (95v/v%) and the lower portion (95v/v%) of the composition is preferably within 10%, more preferably within 5%, and further preferably within 0.5%.

In the composition of the present invention, as described above, the compound represented by the above general formula (1), an isomer thereof and/or a pharmacologically acceptable salt thereof can exert an effect of preventing or improving wrinkle formation. Moreover, the compound in the composition of the present invention is excellent in decomposition stability and dispersion stability over time. Therefore, the composition of the present invention can be suitably used for skin external preparations, is more preferably applied to cosmetics, and is particularly preferably applied to anti-aging cosmetics. Here, cosmetics used herein include quasi-drugs.

The composition of the present invention can arbitrarily contain, in addition to such components, arbitrary components that are used in general skin external preparations, as long as the advantageous effects of the present invention are not impaired. Examples of such arbitrary components include various active components, oil components, surfactants, polyhydric alcohols, thickeners, and ultraviolet light absorbers.

Examples of an active component include a whitening component, a wrinkle-improving component, an anti-inflammatory component, and extracts derived from animals and plants.

Whitening components are not particularly limited, as long as these components are generally used in cosmetics. Examples thereof include 4-n-butyl resorcinol, ascorbic acid glucoside, 3-O-ethyl ascorbic acid, tranexamic acid, albutin, ellagic acid, kojic acid, linoleic acid, nicotinic acid amide, 5,5'-dipropylbiphenyl-2, 2'-diol, disodium 5'-adenylate, cetyl tranexamate, potassium salt of 4-methoxysalicylic acid, hydroquinone, and pantothenic acid.

Examples of other wrinkle improving components include, but are not particularly limited to, as long as they are generally used for cosmetics, nicotinic acid amide, vitamin A or a derivative thereof (retinol, retinal, retinoic acid, tretinoin, isotretinoin, tocopherol retinoate, retinol palmitate, retinol acetate, etc.), ursolic acid benzyl ester, ursolic acid phosphate ester, betulinic acid benzyl ester, and benzyl acid phosphate ester.

Extracts derived from animals and plants are not particularly limited, as long as the extracts are generally used for cosmetics. Preferred examples thereof include a chocolate vine extract, a hiba extract, an asparagus extract, an avocado extract, a sweet hydrangea leaf extract, an almond extract, an arnica extract, an aloe extract, an alonia extract, an apricot extract, a ginkgo extract, an Indian kino tree extract, a fennel extract, an udo extract, an eijitsu rose extract, an *Ezoukogi* (*Eleutherococcus senticosus*) extract, an *Enmeisou* or *Hikiokoshi* (*Isodon japonicus* HARA) extract, a scutellaria root extract, a phellodendron bark extract, O-ren (*Coptis japonica*) extract, a panax extract, an *Otogirisou* (*Hypericum erectum*) extract, an *Odorikosou* (*Lamium*) extract, an orange extract, a *Kakyoku* (*Pyracantha fortuneana*) fruit extract, a pueraria lobata root extract, a chamomilla extract, a *carrot*(*Daucus carota sativa*) extract, a *Kawarayomogi* (*Artemisia capillaris*) extract, a licorice extract, a kiwi extract, a cucumber extract, a guava extract, a *Kujin* (*Sophora angustifolia*) extract, a *Kuchinashi* (*Gardenia florida*) extract, a *Kumazasa* (*Sasa veitchii*) extract, a *Kurara* (*Sophora flavescens*) extract, a walnut extract, a grapefruit extract, a black rice extract, a chlorella extract, a mulberry extract, a *Keikettou* (*Spatholobus suberectus*) extract, a *Gettoyo* (*Alpinia speciose*) extract, a Gentiana (*Gentiana Lutea*) extract, a *Genno-shoko* (*Geranium Thunbergii*) extract, a black tea extract, a burdock extract, a rice extract, a fermented rice extract, a fermented rice bran extract, rice germ oil, a bilberry extract, a salvia extract, a *Sabonsou* (*Saponaria officinalis*) extract, a bamboo grass extract, a hawthorn extract, a *Sansha* (*Bombix mori*) extract, a *Sansho* (*Zanthoxylum Piperitum*) extract, a shiitake mushroom extract, a *Jiou* (*Rehmannia glutinosa*) extract, a *Shikon* (*Lithospermum erythrorhizon*) extract, a *Shiso* (*Perilla ocymoides*) extract, a linden extract, a *Shimotsukesou* (*Spiraea ulmaria*) extract, a peony extract, a *Shoukyo* (*Zingiber officinale*) extract, a *Shoubu kon* (*Acorus calamus*) root extract, a silver burch (*Betula alba* etc.,) extract, a *Sugina* (*Equisetum arvense*) extract, a stevia extract, fermented stevia, an ivy (*Hedera helix*) extract, a *Seiyosanzashi* (*Crataegus monogyna*) extract, a *Seiyoniwatoko* (*Sambucus nigra*) extract, a *Seiyonokogiri* (*Achillea millefolium*) extract, a peppermint (*Mentha piperita*) extract, a sage extract, a mallow (*Malva sylvestris*) extract, a *Senkyu* (*Cnidium officinale*) extract, a *Senburi* (*Swertia japonica*) extract, a *Souhakuhi* (*Morus alba* etc.,) extract, a rhubarb extract, a soybean extract, a *Taisou* (*Zizyphus jujube* etc.,) extract, a thyme extract, a dandelion extract, a tea extract, a clove (*Eugenia caryophyllus*) extract, a citrus unshiu peel extract, a *Tencha* (*Rubus suavissimus* (Raspberry)) extract, a red pepper extract, a Touki (*Angelica acutiloba*) extract, a *Toukinsenka* (*Calendula officinalis*) extract, a peach kernel extract, a *Touhi* (*Citrus aurantium* or *Citrus aurantium daidai*) extract, a *Dokudami* (*Houttuynia cordata*) extract, a tomato extract, a fermented soybean extract, a carrot extract, a garlic extract, a *Novara* (*Rosa canina*) extract, a hibiscus extract, a *Bakumondo* (Ophiopogon root) extract, a lotus extract, a parsley extract, a birch extract, an witch hazel extract, a *Hikiokoshi* (*Isodon japonicus*) extract, a cypress extract, a *Biwa* (*Eriobotrya japonica*) extract, a *Fukitanpopo* (*Tussilago farfara*) extract, a *Fukinotou* (flower stalk of giant butterbur) extract, a poria sclerotium extract, a butcher's broom extract, a grape extract, a grape seed extract, a loofah extract, a safflower extract, a peppermint extract, a linden extract, a peony extract, a hop extract, a pine extract, a marjoram extract, a horse chestnut extract, an Asian skunk cabbage extract, an Indian soapberry extract, a lemon balm extract, a namacystus extract, a peach extract, a cornflower extract, an Eucalyptus extract, a strawberry geranium extract, a yuzu extract, a lily extract, a coix seed extract, a mugwort extract, a lavender extract, a green tea extract, an apple extract, a rooibos tea extract, a lychee extract, a lettuce extract, a lemon extract, a golden bells extract, a Chinese milk vetch extract, a rose extract, a rosemary extract, a Roman chamomile extract, a royal jelly extract, and a great burnet extract.

Examples of the anti-inflammatory component include kurarinone, glabridin, glycyrrhizic acid, glycyrrhetinic acid, and pantothenyl alcohol, and preferred examples thereof are glycyrrhizinic acid and its salt, alkyl glycyrrhetinate and its salt, and glycyrrhetinic acid and its salt.

Examples of surfactants include anionic surfactants such as fatty acid soap (sodium laurate, sodium palmitate etc.), potassium lauryl sulfate, and alkyl sulfuric acid triethanolamine ether, cationic surfactants such as stearyltrimethylammonium chloride, benzalkonium chloride, and laurylamine oxide, amphoteric surfactants such as betaine surfactants (alkyl betaine, amido betaine, sulfobetaine etc.), imidazoline amphoteric surfactants (2-cocoyl-2-imidazolinium hydroxide-1-carboxyethyloxy-2-sodium salt, etc.), and acyl methyl taurine, and nonionic surfactants such as polyglyceryl sesquiisostearate, sorbitan fatty acid esters (sorbitan monostearate, sorbitan sesquioleate, etc.), glycerine fatty acid esters (glyceryl monostearate etc.), propylene glycol fatty acid esters (propylene glycol monostearate etc.), hardened castor oil derivative, glycerin alkyl ether, POE sorbitan fatty acid esters (POE sorbitan monooleate, polyoxyethylene sorbitan monostearate etc.), POE sorbit fatty acid esters (POE-sorbit monolaurate etc.), POE glycerin fatty acid esters (POE-glycerin monoisostearate etc.), POE fatty acid esters (polyethylene glycol monooleate, POE distearate etc.), POE alkyl ethers (POE2-octyl dodecyl ether etc.), POE alkyl phenyl ethers (POE nonyl phenyl ether etc.), pluronic-type surfactants, POE·POP alkyl ethers (POE·POP 2-decyl tetradecyl ether etc.), tetronic surfactants, POE castor oil·hardened castor oil derivatives (POE castor oil, POE hardened castor oil etc.), sucrose fatty acid ester, and alkyl glucoside.

Examples of polyhydric alcohols include polyethylene glycol, glycerin, 1,3-butylene glycol, erythritol, sorbitol, xylitol, maltitol, propylene glycol, dipropylene glycol, diglycerin, isoprene glycol, 1,2-pentanediol, 2,4-hexylene glycol, 1,2-hexanediol, and 1,2-octanediol.

Examples of thickeners include, guar gum, quince seed, carrageenan, galactan, gum arabic, pectin, mannan, starch, xanthan gum, curdlan, methyl cellulose, hydroxyethyl cellulose, carboxymethyl cellulose, methyl hydroxypropyl cellulose, chondroitin sulfate, dermatan sulfate, glycogen, heparan sulfate, hyaluronic acid, sodium hyaluronate, gum traganth, keratan sulfate, chondroitin, mucoitin sulfate, hydroxyethyl guar gum, carboxymethyl guar gum, dextran, kerato sulfate, locust bean gum, succinoglucan, charonic acid, chitin, chitosan, carboxymethyl chitin, agar, polyvinyl alcohol, polyvinyl pyrrolidone, carboxyvinyl polymer, alkyl modified carboxyvinyl polymer, sodium polyacrylate, polyethylene glycol, and bentonite.

Examples of UV absorbers include a para-aminobenzoic acid UV absorber, an anthranilic acid UV absorber, a salicylic acid UV absorber, a cinnamic acid UV absorber, a benzophenone UV absorber, a sugar UV absorber, and UV absorbers such as a 2-(2'-hydroxy-5'-t-octylphenyl)benzotriazole and 4-methoxy-4'-t-butyldibenzoylmethane.

The composition of the present invention can be produced by treating the above essential components, preferred components, arbitrary components and the like according to a conventional method.

### EXAMPLES

The present invention will be described in detail below with reference to Examples, but is of course not limited to the following Examples.

### <Production Example 1>

According to the formulations shown in Tables 1 and 2 below, skin external preparations in the form of oily gelling agent were produced. Specifically, components (i) were dissolved by heating at 95°C and then mixed. Further, components (ii) were added to the mixture while stirring with a disper at 3000 rpm for 1 minute at 95°C. Subsequently, degassing was performed, and then containers were filled with the resultants, followed by solidification by cooling to obtain oil-based solid cosmetics.

### <Test example>

### (1) Evaluation of decomposition stability of KSK32

The content of KSK32 after 2 weeks of storing of each prepared oil-based solid cosmetic at 60°C was measured, and then the proportion (residual rate) thereof was calculated relative to the content of KSK32 after 2 weeks of storing at 5°C designated as 100. Stability to decomposition was evaluated as "Excellent" when the residual rate was 95% or more, "Good" when the same was less than 95% and 80% or more, "Average" when the same was less than 80% and 60% or more, and "Poor" when the same was less than 60%. The results are shown together in Tables 1 and 2.

Note that the content of KSK32 was measured by weighing about 0.5 g of a cosmetic, diluting the cosmetic with a mobile phase in such a manner that the amount was 50 mL to prepare a sample solution, and then subjecting the sample solution to high performance liquid chromatography (HPLC).

The HPLC conditions were as follows: column; reverse phase system column (3.0 × 100 mm), column temperature; room temperature, mobile phase; aqueous solution of anionic sulfonic acid surfactant/THF 25%, pH; 3, flow rate; 0.4 mL/min., detection; 240 nm).

### (2) Evaluation of dispersion stability

After each of the prepared oil-based solid cosmetics was left to stand at 40°C for 1 day, the presence or absence of KSK32 sedimentation was visually observed. When it was uniformly dispersed, the dispersion stability was evaluated as "Good", and when sedimentation was observed, the dispersion stability was evaluated as "Poor". The results are shown together in Tables 1 and 2.

**Table 1 (mass%)**

| Component 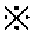 | | Example 1 | Example 2 | Example 3 | Example 4 | Example 5 | Example 6 | Example 7 | Example 8 | Example 9 | Example 10 |
|---|---|---|---|---|---|---|---|---|---|---|---|
| i | Ttriethylhexanoin(0) | 69.45 | 67.45 | 71.95 | - | - | - | - | 69.45 | 88.45 | 51.45 |
| | Squalene(0) | - | - | - | 69.45 | - | - | - | - | - | - |
| | Dimethicone(0) | - | - | - | - | 69.45 | - | - | - | - | - |
| | Hydrogenated polydecene(0) | - | - | - | - | - | 69.45 | - | - | - | - |
| | Cetyl ethylhexanoate(0) | - | - | - | - | - | - | 69.45 | - | - | - |
| | Glyceryl triisostearate(0) | - | - | - | - | | - | - | - | - | - |
| | Polyglyceryl monoisostearate-2(3) | | | - | - | - | - | - | - | - | - |
| | Microcrystalline wax | 22.00 | 22.00 | 22.00 | 22.00 | 22.00 | 22.00 | 22.00 | 22.00 | 3.00 | 40.00 |
| | Polyethylene | - | - | - | - | - | - | - | - | - | - |
| | Paraffin | - | - | - | - | - | - | - | - | - | - |
| | Vaseline | 5.00 | 5.00 | 5.00 | 5.00 | 5.00 | 5.00 | 5.00 | 5.00 | 5.00 | 5.00 |
| | Dextrin palmitate | - | - | - | - | - | - | - | - | - | - |
| ii | Polyglyceryl sesquiisostearate | 0.50 | 0.50 | 0.50 | 0.50 | 0.50 | 0.50 | 0.50 | 0.50 | 0.50 | 0.50 |
| | KSK32 | 0.05 | 0.05 | 0.05 | 0.05 | 0.05 | 0.05 | 0.05 | 0.05 | 0.05 | 0.05 |
| | Porous silica | 3.00 | 5.00 | 0.50 | 3.00 | 3.00 | 3.00 | 3.00 | 3.00 | 3.00 | 3.00 |
| | Kaoline | - | - | - | - | - | - | - | - | - | - |
| Total | | 100.00 | 100.00 | 100.00 | 100.00 | 100.00 | 100.00 | 100.00 | 100.00 | 100.00 | 100.00 |
| KSK32 residual rate | | Excellent | Excellent | Excellent | Excellent | Excellent | Excellent | Excellent | Excellent | Excellent | Excellent |
| KSK32 dispersion stability | | Good | Good | Good | Good | Good | Good | Good | Good | Good | Good |

| | | | | | | | | | | | |
|---|---|---|---|---|---|---|---|---|---|---|---|
| 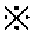 Numerical figures in parentheses following the name of each oiling agent indicate the number of hydroxy groups. | | | | | | | | | | | |

**Table 2 (mass%)**

| Component 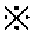 | | Example 11 | Example 12 | Example 13 | Example 14 | Example 15 | Example 16 | Example 17 | Example 18 | Example 19 | Comparative example 1 |
|---|---|---|---|---|---|---|---|---|---|---|---|
| i | Ttriethylhexanoin(0) | 69.45 | 69.45 | 67.45 | 52.45 | 69.45 | 89.45 | 49.45 | 24.45 | - | - |
| | Squalene(0) | - | - | - | - | - | - | - | - | - | - |
| | Dimethicone(0) | - | - | - | - | - | - | - | - | - | - |
| | Hydrogenated polydecene(0) | - | - | - | - | - | - | - | - | - | - |
| | Cetyl ethylhexanoate(0) | - | - | - | - | - | - | - | - | - | - |
| | Glyceryl triisostearate(0) | - | - | - | - | - | - | 20.00 | 45.00 | 69.00 | - |
| | Polyglyceryl monoisostearate-2(3) | - | - | - | - | - | - | - | - | - | 69.00 |
| | Microcrystalline wax | - | - | 22.00 | 22.00 | 25.00 | - | 22.00 | 22.00 | 22.00 | - |
| | Polyethylene | 22.00 | - | - | - | - | - | - | - | - | - |
| | Paraffin | - | 22.00 | - | - | - | - | - | - | - | - |
| | Vaseline | 5.00 | 5.00 | 5.00 | 5.00 | 5.00 | 5.00 | 5.00 | 5.00 | 5.00 | - |
| | Dextrin palmitate | - | - | - | - | - | - | - | - | - | 9.00 |
| ii | Polyglyceryl sesquiisostearate | 0.50 | 0.50 | 0.50 | 0.50 | 0.50 | 0.50 | 0.50 | 0.50 | 0.50 | - |
| | KSK32 | 0.05 | 0.05 | 0.05 | 0.05 | 0.05 | 0.05 | 0.05 | 0.05 | 0.05 | 0.05 |
| | Porous silica | 3.00 | 3.00 | - | - | - | 5.00 | 3.00 | 3.00 | 3.00 | 3.00 |
| | Kaoline | - | - | 5.00 | 20.00 | - | - | - | - | - | - |
| Total | | 100.00 | 100.00 | 100.00 | 100.00 | 100.00 | 100.00 | 100.00 | 100.00 | 99.55 | 81.05 |
| KSK32 residual rate | | Excellent | Excellent | Excellent | Excellent | Excellent | Excellent | Excellent | Good | Average | Poor |
| KSK32 dispersion stability | | Good | Good | Good | Good | Poor | Poor | Good | Good | Good | Good |

| | | | | | | | | | | | |
|---|---|---|---|---|---|---|---|---|---|---|---|
| 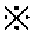 Numerical figures in parentheses following the name of each oiling agent indicate the number of hydroxy groups. | | | | | | | | | | | |

### <Production Example 2>

According to the formulation shown in Table 3 below, a skin external preparation in the dosage form of cream was prepared, and according to the formulation shown in Table 4, a skin external preparation in the dosage form of milk was prepared. Specifically, components (i) were dissolved by heating at 95°C and then mixed. Further, components (ii) were added to the mixture while stirring with a disper at 3000 rpm for 1 minute at 95°C to obtain each oil-based cosmetic.

It was also confirmed by the method in the above test example that the oil-based cosmetics of Examples 20 and 21 were excellent in decomposition stability and dispersion stability of KSK32.

**Table 3 (mass%)**

| Component 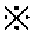 | | Example 20 |
|---|---|---|
| i | Ttriethylhexanoin(0) | 57.00 |
| | Cetyl 2-ethylhexanoate(0) | 5.00 |
| | Dextrin (palmitate/octanoate) | 3.00 |
| | Dextrin palmitate | 7.00 |
| ii | KSK32 | 3.00 |
| | (Vinyl dimethicone/methicone silsesquioxane) crosspolymer (spherical) | 20.00 |
| | Porous silica (spherical) | 5.00 |
| Total | | 100.00 |

| | | |
|---|---|---|
| 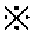 Numerical figures in parentheses following the name of each oiling agent indicate the number of hydroxy groups. | | |

**Table 4 (mass%)**

| Component 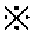 | | Example 21 |
|---|---|---|
| i | Ttriethylhexanoin(0) | 5.00 |
| | Squalene(0) | 88.00 |
| | Dextrin (palmitate/octanoate) | 2.00 |
| | Dextrin palmitate | 2.00 |
| ii | KSK32 | 3.00 |
| Total | | 100.00 |

| | | |
|---|---|---|
| 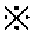 Numerical figures in parentheses following the name of each oiling agent indicate the number of hydroxy groups. | | |

### INDUSTRIAL APPLICABILITY

The present invention is applicable to skin external preparations such as cosmetics.

## Claims

1. An oil-based composition, comprising a compound represented by the following general formula (1), an isomer thereof and/or a pharmacologically acceptable salt thereof and an oiling agent having 0 to 2 hydroxy groups, 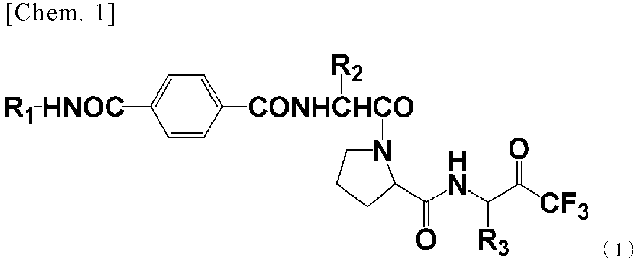 [wherein R₁ represents a linear or branched C1-4 alkyl group substituted with a carboxyl group, or a linear or branched C1-4 alkyl group substituted with a carboxylic acid ester group having a C1-4 alkyl chain, and R₂ and R₃ each independently represent a linear or branched C1-4 alkyl group.].

2. The composition according to claim 1, wherein the oiling agent having 0 to 2 hydroxy groups is selected from the group consisting of hydrocarbon oil, silicone oil, and ester oil.

3. The composition according to claim 1 or 2, wherein the content of the oiling agent having 0 to 2 hydroxy groups is 50 mass% or more of the whole composition.

4. The composition according to any one of claims 1 to 3, further comprising an oily gelling agent.

5. The composition according to any one of claims 1 to 4, further comprising a powder.

6. The composition according to claim 5, wherein the content of the powder ranges from 0.5 mass% to 25 mass% of the whole composition.

7. The composition according to any one of claims 1 to 6, which is in a form of oily gelling agent.

8. The composition according to claim 7, comprising a solid fat and/or a semi-solid fat in an amount of 5 mass% or more of the whole composition.

9. The composition according to any one of claims 1 to 8, which substantially contains no water.

10. The composition according to any one of claims 1 to 9, which is a skin external preparation.
